# EUROPEAN PATENT SPECIFICATION

(11) **EP 1 854 456 B1**
(45) Date of publication and mention of the grant of the patent: **23.08.2017**
(21) Application number: 06714680.3
(22) Date of filing: 27.02.2006
(51) Int. Cl.: A61K 9/70, A61K 47/02, A61K 47/14, A61K 47/32

(54) **PRESSURE-SENSITIVE ADHESIVE BASE AND MEDICAL ADHESIVE PATCH INCLUDING THE PRESSURE-SENSITIVE ADHESIVE BASE**
HAFTKLEBSTOFFBASIS UND MEDIZINISCHES KLEBEPFLASTER MIT DER HAFTKLEBSTOFFBASIS
BASE ADHÉSIVE SENSIBLE À LA PRESSION ET PATCH ADHÉSIF MÉDICAL CONTENANT LA BASE ADHÉSIVE SENSIBLE À LA PRESSION

(30) Priority: 28.02.2005 JP 2005053534
(43) Date of publication of application: 14.11.2007
(73) Proprietor: Hisamitsu Pharmaceutical Co., Inc., Tosu-shi, Saga 841-0017 (JP)
(72) Inventor: YASUKOCHI,Takashi Hisamitsu Pharmaceutical Co., Inc., Tsukuba-shi, Ibaraki 3050856 (JP); HONMA, Sachiko Hisamitsu Pharmaceutical Co., Inc., Tsukuba-shi, Ibaraki, 3050856 (JP); TATEISHI, Tetsuro Hisamitsu Pharmaceutical Co., Inc., Tsukuba-shi, Ibaraki, 3050856 (JP)
(74) Representative: Forstmeyer, Dietmar
(86) International application number: PCT/JP2006/303543
(87) International publication number: WO 2006/093066

(56) References cited:
- EP-A1- 1 477 189
- EP-A2- 0 680 754
- DE-A1- 19 609 033
- JP-A- 03 127 728
- JP-A- 05 065 224
- JP-A- 07 304 672
- JP-A- 11 501 323
- JP-A- 2004 026 747

## Description

### Technical Field

The present invention relates to a pressure-sensitive adhesive base and an adhesive patch that contain a crosslinking polymer as a main component.

### Background Art

The so-called percutaneous absorption-type adhesive patches, which administer a drug via the skin, employ a nonaqueous pressure-sensitive adhesive that uses as a base a polymer component containing substantially no water since a drug added to a layer of the pressure-sensitive adhesive (plaster layer) often has a low polarity and is insoluble in water.

The adhesive patches employing a nonaqueous pressure-sensitive adhesive layer sometimes contain in the adhesive layer a liquid organic component that is miscible with the polymer component forming the adhesive layer. This is because; by adding the liquid organic component, the pressure-sensitive adhesive is softened so as to improve its conformability to the skin, thus guaranteeing the actual attachment area, and the peel-off force when the patch is peeled off from the skin is reduced, thus suppressing the pain upon peeling-off and the subsequent skin irritation; and, in addition, because such a liquid organic component often acts on the skin to enhance the skin permeability of the drug. It could therefore be very important to add to the adhesive patch a liquid organic component in a large amount with such combination of useful functions in order to realize an adhesive patch having good applicability and permeability toward the skin.

However, addition of a large amount of a liquid organic component to the pressure-sensitive adhesive layer disturbs the balance between adhesive strength and cohesive strength and causes new problems such as cohesive failure and stringiness of the pressure-sensitive adhesive layer. In order to solve these problems, a technique to improve the cohesive strength of the pressure-sensitive adhesive layer by crosslinking the polymer forming the base has conventionally been employed (ref. Patent Publication 1). This crosslinking of the polymer may be carried out in advance, however, for ease of molding, etc., it is often carried out simultaneously at the time of production of an adhesive patch. For example, since isocyanate and metal alcoholate are readily soluble in an organic solvent and the reaction proceeds quickly, they have often been used for crosslinking of a nonaqueous polymer containing a carboxyl group or a hydroxyl group. However, since these compounds are highly reactive, there is the possibility of drug decomposition, skin irritation, etc. and, furthermore, there are the problems of the difficulties in controling the reaction rate due to the compounds being readily soluble in the organic solvent, and the difficulties in molding due to the solidification of pressure-sensitive adhesive layer during production.

On the other hand, an attempt has been made to form a soft pressure-sensitive adhesive from a conventional acrylic pressure-sensitive adhesive composition by adding a large amount of an organic liquid component that is miscible with an acrylic copolymer so as to weaken the strong bond between copolymer molecules (ref. Patent Publications 2 and 3). It was found that improvements were made such that stripping of the stratum corneum upon peeling-off of the adhesive patch from the skin was suppressed, and that the occurrence of physical irritation was inhibited.

However, when an oxide or hydroxide of a multivalent ionic metal, which does not dissolve in the copolymer solution, is used as a crosslinking agent in such acrylic pressure-sensitive adhesive composition, stable physical properties cannot be obtained, since the crosslinking reaction gradually proceeds and the pressure-sensitive adhesive layer solidifies in a few days to a few weeks. On the other hand, it is possible to increase the apparent reaction rate by increasing the added amount of the oxide/hydroxide required for the crosslinking reaction to suppress the change in physical properties over time, however, the oxide and hydroxide, which are insoluble components, cause problems such as an insufficient adhesive strength, and excessive crosslinking progression, thus causing exudation (bleeding) of the liquid component from the patch.

Patent Publication 1: Japanese Patent No. 2967788
Patent Publication 2: Japanese Patent No. 2700835
Patent Publication 3: JP, A, H03-127728

### Disclosure of Invention

### Problems to be Solved by the Invention

The object of the present invention is therefore to provide a pressure-sensitive adhesive base that has appropriate adhesion physical properties (adhesive strength and cohesive strength) while containing the above-mentioned liquid organic component, causes no bleeding and very little damage to the skin corneum, and has suppressed skin irritation, and to provide an adhesive patch containing such pressure-sensitive adhesive base.

### Means for Solving the Problems

During the course of an intensive investigation by the inventors trying to solve the above-mentioned problems, it has been found for the first time that, surprisingly, using a small amount of a multivalent metal oxide or hydroxide as a crosslinking agent for a carboxyl group-containing nonaqueous polymer enables to form a crosslinking polymer pressure-sensitive adhesive base containing a relatively high concentration of a liquid or semisolid organic component, which has conventionally been considered to be difficult; it has also been found that an adhesive patch employing such pressure-sensitive adhesive base has an appropriate adhesive strength and cohesive strength, causes very little damage to the skin corneum, causes no bleeding, and has excellent physical properties and usability, and the present invention has thus been accomplished.

Namely, the present invention relates to a pressure-sensitive adhesive base comprising components A to C below;
A. a copolymer having as monomer units a carboxyl group-containing monomer and a (meth)acrylic acid ester,
B. one or more type of liquid or semisolid organic compound that is miscible with the copolymer of component A, and
C. at least one type selected from a multivalent metal oxide and a multivalent metal hydroxide,
the total content of component C being 0.001% to 5% of the total mass of the pressure-sensitive adhesive base, and the base containing substantially no water.

The present invention also relates to the pressure-sensitive adhesive base wherein it is produced via a step of dispersing and/or dissolving component C in a solvent that does not contain components A and B.

Moreover, the present invention relates to the pressure-sensitive adhesive base wherein the total content of component B is at least 10% of the total mass of the pressure-sensitive adhesive base.

Furthermore, the present invention relates to a medical adhesive patch comprising a drug and the pressure-sensitive adhesive base.

Namely, the present invention enables the cohesiveness of a pressure-sensitive adhesive to be adjusted and a pressure-sensitive adhesive base to have both an appropriate adhesive strength and cohesive strength by combining a small amount of a multivalent metal oxide or multivalent metal hydroxide with a copolymer having as monomer units a carboxyl group-containing monomer and a (meth)acrylic acid ester and, moreover, enables to obtain a pressure-sensitive adhesive base with suppressed damaging to the skin corneum and an extremely low skin irritancy by adding a liquid or semisolid organic component. Furthermore, by using a copolymer comprising a carboxyl group-containing monomer, it is enabled to realize more sufficiently strong crosslinking than by using a copolymer with a hydroxyl group-containing monomer, etc. Moreover, by using a multivalent metal oxide or a multivalent metal hydroxide as a crosslinking agent it is enabled to provide better safety to skin with less skin irritation and no drug decomposition, etc. than by using a metal alcoholate, an isocyanate, etc.

In this way, by using a multivalent metal oxide or hydroxide as a crosslinking agent and a carboxyl group-containing polymer as a base while including a liquid or semisolid organic component it is possible to develop a medical adhesive patch that has excellent physical properties as a patch and an excellent usability with good penetration performance.

### Effects of the Invention

In accordance with the present invention, it is possible to provide a pressure-sensitive adhesive base that has both appropriate adhesive strength and appropriate cohesive strength and has appropriate flexibility, and for which damage to the corneum of the skin is remarkably reduced. Since the adhesive patch employing the pressure-sensitive adhesive base of the present invention employs a carboxyl group-containing nonaqueous polymer, it is suitable as a medical adhesive patch to which a lipophilic drug is added. In addition, the adhesive patch employing the pressure-sensitive adhesive base of the present invention is useful because it has a good balance between adhesiveness and cohesiveness, does not cause bleeding, causes remarkably little skin irritation because it strips hardly any corneum when peeled off after its application, and is excellent in terms of skin permeability of the drug.

### Best Mode for Carrying Out the Invention

Preferred modes for carrying out the present invention are explained in detail below.

The copolymer having a carboxyl group-containing monomer and a (meth)acrylic acid ester as monomer units, which is used in the pressure-sensitive adhesive base of the present invention, is not particularly limited, but acrylic polymers and methacrylic polymers are suitably used.

Such acrylic and methacrylic polymers are not particularly limited; those containing acrylic acid or methacrylic acid as the carboxyl group-containing monomer are preferable and, as the (meth)acrylic acid ester, one or more types of monomers selected from the group consisting of acrylic and methacrylic acid straight-chain alkyl esters such as methyl, ethyl, propyl, butyl, pentyl, hexyl, heptyl, octyl, nonyl, decyl, undecyl, dodecyl, and tridecyl esters, branched alkyl esters such as a 2-ethylhexyl ester, and substituted alkyl esters such as 2-hydroxyethyl, 3-hydroxypropyl, and 4-hydroxybutyl esters can be used.

The carboxyl group-containing monomer is preferably 5 to 50 mass % of the copolymer of component A and, when the drug that is formulated is basic, it is more preferably 5 to 25 mass % so as not to degrade the release characteristics thereof.

The polymer contained in the pressure-sensitive adhesive base of the present invention may contain, in addition to the carboxyl group-containing monomer and the (meth)acrylic acid ester, one or more kinds of other monomers. Examples of such monomers include acrylonitrile, vinyl acetate, *N*-vinyl-2-pyrrolidone, itaconic acid, maleic acid, allylamine, styrene, reactive polymers (macromonomers), vinyl propionate, methylvinylpyrrolidone, vinylpyridine, vinylpiperidone, vinylpiperazine, vinylpyrrole, vinylimidazole, vinylcaprolactam, vinyloxazole, vinylmorpholine, 2-ethylhexyl acrylate, vinylpyrrolidone, methoxyethyl acrylate, and acrylic acid, and vinyl acetate, 2-ethylhexyl acrylate, and vinylpyrrolidone are particularly preferable.

Specific examples of the copolymer of component A, which is used in the pressure-sensitive adhesive base of the present invention, include (acrylic acid-2-ethylhexyl acrylate) copolymer, (acrylic acid-butyl acrylate) copolymer, (acrylic acid-octyl acrylate) copolymer, (acrylic acid-2-ethylhexyl acrylate-vinyl acetate) copolymer, (acrylic acid-2-ethylhexyl acrylate-hydroxyethyl acrylate) copolymer, (acrylic acid-hydroxyethyl acrylate-vinylpyrrolidone) copolymer, and (acrylic acid-2-ethylhexyl acrylate-hydroxyethyl acrylate-vinylpyrrolidone) copolymer, and it is also possible to use a commercial product such as the DURO-TAK (registered trademark) series (manufactured by National Starch and Chemical Company) or the Gelva (registered trademark) multipolymer solution series (manufactured by Monsanto Company).

The pressure-sensitive adhesive base of the present invention may employ one type of copolymer consists of the above-mentioned monomers on its own, or two or more types in combination.

The content of the copolymer having such a carboxyl group-containing monomer and (meth)acrylic acid ester as monomer units is not particularly limited, and is preferably 20 to 90 mass % relative to the mass of the entire composition of the pressure-sensitive adhesive base, more preferably 40 to 80 mass %, and particularly preferably 50 to 70 mass %. When the content is less than 20 mass %, the cohesive strength is insufficient and the plaster tends to remain on the skin or cause stringiness, and when it exceeds 90 mass % the adhesive strength tends to become too high and strip the corneum, etc. when the patch is peeled off and cause skin irritation.

A liquid or semisolid organic compound that is miscible with the copolymer of component A used in the pressure-sensitive adhesive base of the present invention may be an absorption promoting agent, a solubilizing agent, a plasticizer, etc. miscible with the copolymer of component A.

The absorption promoting agent that can be used here can be any compound that is conventionally recognized to have a skin absorption-promoting effect, including, for examples, such as fatty acids, fatty alcohols, and fatty acid esters, and ethers having 6 to 20 carbons, aromatic organic acids, aromatic alcohols, aromatic organic acid esters and ethers (those above can be either saturated or unsaturated, and can be cyclic, straight chain, or branched) and, furthermore, lactate esters, acetate esters, monoterpenoid compounds, Azone, Azone derivatives, glycerol fatty acid esters, sorbitan fatty acid esters (Span series), polysorbate types (Tween series), polyethylene glycol fatty acid esters, polyoxyethylene hardened castor oil types (HCO series), sugar fatty acid esters, and fatty acid alkylolamides.

Specifically, preferred examples include caprylic acid, capric acid, caproic acid, lauric acid, myristic acid, palmitic acid, stearic acid, oleic acid, linoleic acid, linolenic acid, lauryl alcohol, myristyl alcohol, oleyl alcohol, cetyl alcohol, methyl laurate, isopropyl myristate, myristyl myristate, octyldodecyl myristate, cetyl palmitate, salicylic acid, methyl salicylate, ethylene glycol salicylate, cinnamic acid, methyl cinnamate, cresol, cetyl lactate, ethyl lactate, propyl lactate, myristyl lactate, geraniol, thymol, eugenol, terpineol, 1-menthol, borneol, d-limonene, isoeugenol, isoborneol, nerol, dl-camphor, glyceryl monolaurate, sucrose monolaurate, polysorbate 20, propylene glycol, dipropylene glycol, polyethylene glycol monolaurate, polyethylene glycol monostearate, HCO-60, and 1-[2-(decylthio)ethyl]azacyclopentan-2-one (hereinafter, abbreviated to 'pyrrothiodecane'), and particularly preferred examples include lauryl alcohol, 1-menthol, propylene glycol, pyrrothiodecane, dipropylene glycol, isopropyl myristate, and lauric diethanolamide.

Examples of the plasticizer used in the present invention include paraffin oil, squalane, squalene, plant oils (e.g., olive oil, camellia oil, castor oil, tall oil, peanut oil), dibasic acid esters (e.g., dibutyl phthalate, dioctyl phthalate, etc.), liquid rubber (e.g., polybutene, liquid isoprene rubber), diethylene glycol, polyethylene glycol, glycol salicylate, propylene glycol, dipropylene glycol, and crotamiton. In particular, liquid paraffin, liquid polybutene, glycol salicylate, and crotamiton are preferable.

Examples of the solubilizing agent include dipropylene glycol, glycerol, ethylene glycol, and polyethylene glycol.

Such a liquid or semisolid organic compound that is miscible with the copolymer of component A may be used alone or in combination of two or more thereof. The content of the liquid or semisolid organic compound in the pressure-sensitive adhesive base of the present invention is not particularly limited, and is preferably at least 10 mass % relative to the mass of the entire pressure-sensitive adhesive base composition, and more preferably 10 to 80 mass %. When it is less than 10 mass %, the adhesive strength tends to become too high and strip the corneum, etc. when the patch is peeled off, and then cause skin irritation, and when it exceeds 80 mass %, the cohesive strength is insufficient and the plaster tends to remain on the skin or cause stringiness.

The multivalent metal oxide or multivalent metal hydroxide used as a crosslinking agent in the present invention is not particularly limited; examples thereof may include oxides or hydroxides of calcium, magnesium, aluminum, zinc, titanium, etc., and the compounds thereof may include calcium oxide, calcium hydroxide, magnesium oxide, magnesium hydroxide, aluminum oxide, aluminum hydroxide, zinc oxide, and titanium oxide. These multivalent metal oxides and multivalent metal hydroxides may be used alone or in combination of two or more thereof.

The content of the multivalent metal oxide or multivalent metal hydroxide in the pressure-sensitive adhesive base of the present invention is 0.001 to 5 mass % relative to the mass of the entire composition of the pressure-sensitive adhesive base, more preferably 0.05 to 3 mass %, and particularly preferably 0.1 to 2 mass %. When it is less than 0.001 mass %, crosslinking is inadequate, so that the cohesive strength is insufficient and the plaster tends to remain on the skin or cause stringiness, whereas when it exceeds 5 mass %, the amount of insoluble components increases relative to that of the copolymer, so that the adhesive strength tends to be insufficient, or the crosslinking tends to progress too far, thus causing bleeding.

When the pressure-sensitive adhesive base of the present invention is produced, mixing of the carboxyl group-containing polymer and the crosslinking agent (multivalent metal oxide or multivalent metal hydroxide) is carried out by, for example, dispersing or dissolving the crosslinking agent in advance in a solvent containing a lower alcohol (ethanol, etc.) and adding the liquid in which the crosslinking agent is dispersed or dissolved to a mixture containing the polymer and the liquid or semi-solid organic compound, or by adding the crosslinking agent to a solution containing a lower alcohol and the polymer. Subsequently, the mixture of the polymer and the crosslinking agent thus obtained is molded into any desired shape such as a sheet, a tape, or a rod, and then thermally crosslinked. The thermal crosslinking can also be carried out simultaneously with the molding, as in a case where the mixture is poured into a heated mold.

The lower alcohol is preferably a monovalent alcohol having 1 to 5 carbons, due to the ease of their removal from the patch during drying step, and in order to carry out a simultaneous thermal crosslinking reaction, the lower alcohol is preferably methanol (boiling point 65°C), ethanol (boiling point 78°C), or isopropanol (boiling point 83°C), which have a boiling point at 90° C or lower, of those ethanol is used with particular preference.

There is no need for using water in the production of the pressure-sensitive adhesive base of the present invention; or even if water is used it can be removed by heating. Therefore, it is possible to obtain a pressure-sensitive adhesive base containing substantially no water.

Herein, 'containing substantially no water' means that no water is used in the production of the pressure-sensitive adhesive base, or the pressure-sensitive adhesive base produced contains no water.

The pressure-sensitive adhesive base of the present invention are not particularly limited in its application; it can be molded into any shape according to its use, and may typically used for medical adhesive patches, plasters, adhesive packaging tape, masking tape/sheet, and insulating tape.

A pressure-sensitive adhesive layer of the medical adhesive patch of the present invention may contain a drug as appropriate, in addition to the above-mentioned pressure-sensitive adhesive base components. Such drug is not particularly limited as long as it can percutaneously permeate a biological membrane, and examples thereof may include general anesthetics, hypnotic/analgesic agents, antipyretic/anti-inflammatory analgesics, steroidal anti-inflammatory agents, analeptics/stimulants, antivertigo agents, agents for psychoneurosis, local anesthetics, skeletal muscle relaxants, agents for the autonomic nervous system, antispasmodics, antiparkinsonian drugs, antihistamines, cardiotonics, antiarrhythmic agents, diuretics, antihypertensives, vasoconstrictors, vasodilators, agents for arteriosclerosis, respiratory stimulants, respiratory stimulants, antitussives/expectorants, agents for treating peptic ulcers, cholagogues, hormonal drugs, agents for urogenital and anal organs, agents for parasitic skin disease, emollients, vitamins, mineral preparations, hemostatics, anticoagulants, agents for liver disease, agents for habitual intoxication, agents for treating gout, agents for diabetes, antineoplastic agents, radioactive drugs, traditional Chinese preparations, antibiotics, chemotherapeutics, anthelmintic/antiprotozoal agents, and narcotics.

Examples of the antipyretic/anti-inflammatory analgesics include acetoaminophenone, phenacetin, mefenamic acid, diclofenac, fulfenamic acid, aspirin, salicylic acid, aminopyrine, alclofenac, ibuprofen, naproxen, flurbiprofen, ketoprofen, sodium amfenac, mepirizole, indomethacin, pentazocine, and piroxicam; and examples of the steroidal anti-inflammatory agents include hydrocortisone, triamcinolone, dexamethasone, betamethasone, and prednisolone.

Examples of the vasodilators include diltiazem, pentaerythritol, isosorbide, trapidil, nicorandil, nitroglycerin, prenylamine, molsidomine, and tolazoline; examples of the antiarrhythmic agents include procainamide, lidocaine, propranolol, alprenolol, atenolol, nadolol, metoprolol, ajmaline, disopyramide, and mexitilen; and examples of the antihypertensives include ecarazine, indapamide, clonidine, bunitrolol, labetalol, captopril, guanabenz, mebutamate, and betanidine.

Examples of the antitussives/expectorants include carbetapentane, cloperastine, oxeladin, clobutinol, clofedanol, noscapine, ephedrine, isoproterenol, clorprenaline, methoxyphenamine, procaterol, tulobuterol, clenbuterol, and ketotifen; examples of the antineoplastic agents include cyclophosphamide, fluorouracil, tegafur, mitomycin C, procarbazine, doxifluridine, and ranimustine; and examples of the local anesthetics include ethyl aminobenzoate, tetracaine, procaine, dibucaine, oxybuprocaine, ambroxol, and propitocaine.

Examples of the hormonal drugs include propylthiouracil, thiamazole, metenolone acetate, estradiol, norethisterone acetate, estriol, and progesterone; examples of the antihistamines include diphenhydramine, chlorpheniramine, promethazine, cyproheptadine, and diphenylpyraline; examples of the anticoagulants include potassium warfarin and ticlopidine; examples of the antispasmodics include methylatropine bromide and scopolamine; examples of the general anesthetics include sodium thiopental and sodium pentobarbital; examples of the hypnotic/analgesic agents include bromvalerylurea, amobarbital, and phenobarbital; examples of anti-epileptics include phenytoin; and examples of the analeptics/stimulants include methamphetamine.

Examples of the antivertigo agents include difenidol and betahistine; examples of the agents for psychoneurosis include chlorpromazine, thioridazine, meprobamate, imipramine, chlordiazepoxide, and diazepam; examples of the skeletal muscle relaxants include suxamethonium and eperisone; examples of the agents for the autonomic nervous system include neostigmine bromide and bethanechol chloride; examples of the antiparkinsonian drugs include pergolide and amantadine; examples of the diuretics include hydroflumethiazide, isosorbide, and furosemide; examples of the vasoconstrictors include phenylephrine; examples of the respiratory stimulants include lobeline, dimorpholamine, and naloxone; and examples of the agents for treating peptic ulcers include glycopyrronium bromide, proglumide, cetraxate, cimetidine, and spizofurone.

Examples of the cholagogues include ursodesoxycholic acid and osalmid; examples of the agents for the urogenital and anal organs include hexamine, sparteine, dinoprost, and ritodrine; examples of the agents for parasitic skin disease include salicylic acid, ciclopiroxolamine, and croconazole; examples of the emollients include urea; examples of the vitamins include calcitriol, thiamine, sodium riboflavin phosphate, pyridoxine, nicotinamide, panthenol, and ascorbic acid; and examples of the hemostatics include ethamsylate.

Examples of the agents for liver disease include tiopronin; examples of the agents for habitual intoxication include cyanamide; examples of the agents for treating gout include colchicine, probenecid, and sulfinpyrazone; examples of the agents for diabetes include tolbutamide, chlorpropamide, sodium glymidine, glybuzole, buformin, and insulin; examples of the antibiotics include benzylpenicillin, propicillin, cloxacillin, ampicillin, bacampicillin, carbenicillin, cephaloridine, cefoxitin, erythromycin, chloramphenicol, tetracycline, kanamycin sulfate, and cycloserine; examples of the chemotherapeutics include isoniazid, pyrazinamide, and ethionamide; and examples of the narcotics include morphine, codeine phosphate, cocaine, fentanyl, and pethidine.

These drugs can be used alone or in combination of two or more thereof, and may be in the form of a pharmaceutically acceptable organic or inorganic salt. The amount of drug added is 0.1 to 30 mass % relative to the mass of the entire composition of the pressure-sensitive adhesive layer while taking into consideration a sufficient skin permeation from the adhesive patch, irritation to the skin, etc.

An example of production of a medical adhesive patch employing the pressure-sensitive adhesive base of the present invention is explained as follows.

The production of the polymer is carried out according to a standard method by appropriately formulating the carboxyl group-containing nonaqueous monomer and any of the above-mentioned various other monomers and polymerizing them. It is also possible to use as a starting material a commercial carboxyl group-containing polymer such as the DURO-TAK (registered trademark) series (manufactured by National Starch and Chemical Company) or the Gelva (registered trademark) multipolymer solution series (manufactured by Monsanto Company).

In order that the copolymer having a carboxyl group-containing monomer and a (meth) acrylic acid ester as monomer units, the liquid or semisolid organic compound, and the crosslinking agent are mixed in the presence of a lower alcohol, the polymer and the liquid or semisolid organic compound are mixed with the lower alcohol in advance, or the crosslinking agent is mixed with the lower alcohol in advance. The mixture thus obtained may further be added with a drug, and addition of the drug is carried out prior to molding, preferably prior to the addition of the crosslinking agent.

Subsequently, the mixture thus obtained is spread on a release film treated with, for example, silicon to be molded into a sheet.

The mixture thus spread and molded into a sheet shape is then heated so as to make a crosslinking reaction proceed. Heating is usually carried out at 60°C to 120°C for about 5 to 30 minutes. When the heating temperature is too high or the heating time is too long, there is a possibility of the drug decomposition/denature. When the heating temperature is too low or the heating time is too short, there is a possibility of the lower alcohol remaining and insufficient crosslinking reaction progressing .

The pressure-sensitive adhesive layer obtained by thermal crosslinking is covered with a support, thus giving a medical adhesive patch employing the pressure-sensitive adhesive base of the present invention.

The present invention is explained more specifically below with reference to the Examples, but the present invention is not to be limited to these Examples and can be modified in a variety of ways as long as the modifications do not depart from the scope and spirit of the present invention. In the Examples, '%' means mass %.

### Examples

### Example 1

**[Table 1]**

| Component name | | Solid content /g | Proportion /% |
|---|---|---|---|
| Acrylic polymer | | 14.88 | 49.6 |
| Isopropyl myristate | | 15 | 50.0 |
| Zinc oxide | | 0.12 | 0.4 |
| Ethanol | For dispersing crosslinking agent | 0.25 | - |
| | For adjusting solid content | 6.12 | - |
| Total | | 30 | 100.0 |

As an acrylic polymer, an (acrylic acid-2-ethylhexyl acrylate-vinyl acetate) copolymer (the content of each monomer in the copolymer being 5 mass % for acrylic acid, 70 mass % for 2-ethylhexyl acrylate, and 25 mass % for vinyl acetate) was used. In the above-indicated composition, the acrylic polymer, isopropyl myristate (Spectrum Chemical, NF), and ethanol for adjusting the solid content were mixed, then zinc oxide (Wako Pure Chemical Industries, Ltd., 0.02 *µ*m) dispersed in ethanol in advance was added thereto, and stirred until the mixture became transparent. The mixture was spread over a silicone-treated surface of an 80 *µ*m thick polyethylene terephthalate (PET) release film, and heated at 80°C for 15 minutes to remove solvent while thermally crosslinking to give a 100 *µ*m pressure-sensitive adhesive layer. As a support, a 30 *µ*m-thick sand matte-treated PET film was superimposed on the pressure-sensitive adhesive layer to be in contact therewith, thus giving an adhesive patch of the present invention.

### Example 2

**[Table 2]**

| Component name | Solid content /g | Proportion /% |
|---|---|---|
| Acrylic polymer | 2.981 | 48.9 |
| Isopropyl myristate | 3.105 | 51.0 |
| Aluminum hydroxide gel 5.7% suspension | 0.0071 | 0.1 |
| Total | 6.0931 | 100.0 |

As an acrylic polymer, an (acrylic acid-2-ethylhexyl acrylate-vinyl acetate) copolymer (the content of each monomer in the copolymer being 5 mass % for acrylic acid, 70 mass % for 2-ethylhexyl acrylate, and 25 mass % for vinyl acetate) was used. In the above-indicated composition, the acrylic polymer and isopropyl myristate (Spectrum Chemical, NF) were mixed prior to the addition of an aluminum hydroxide gel (Tomita Pharmaceutical Co., Ltd., Pharmacopeia of Japan), and stirred until the mixture became transparent. The mixture was spread over a silicone-treated surface of an 80 *µ*m thick polyethylene terephthalate (PET) release film, and heated at 80°C for 15 minutes to remove solvent while thermally crosslinking to give a 100 *µ*m pressure-sensitive adhesive layer. As a support, a 30 *µ*m-thick sand matte-treated PET film was superimposed on the pressure-sensitive adhesive layer to be in contact therewith to give an adhesive patch of the present invention.

### Example 3

**[Table 3]**

| Component name | | Solid content /g | Proportion /% |
|---|---|---|---|
| Acrylic polymer | | 2.485 | 49.7 |
| Isopropyl myristate | | 2.5 | 50.0 |
| Calcium hydroxide | | 0.015 | 0.3 |
| Ethanol | For dispersing crosslinking agent | 0.25 | - |
| | For adjusting solid content | 1.713 | - |
| Total | | 5 | 100.0 |

As an acrylic polymer, an (acrylic acid-2-ethylhexyl acrylate-vinyl acetate) copolymer (the content of each monomer in the copolymer being 5 mass % for acrylic acid, 70 mass % for 2-ethylhexyl acrylate, and 25 mass % for vinyl acetate) was used. In the above-indicated composition, the acrylic polymer, isopropyl myristate (Spectrum Chemical, NF), and ethanol for adjusting the solid content were mixed, then calcium hydroxide (Wako Pure Chemical Industries, Ltd., special grade) dispersed in ethanol in advance was added thereto, and stirred until the mixture became transparent. The mixture was spread over a silicone-treated surface of an 80 *µ*m-thick polyethylene terephthalate (PET) release film, and heated at 80°C for 15 minutes to remove solvent while thermally crosslinking to give a 100 *µ*m pressure-sensitive adhesive layer. As a support, a 30 *µ*m-thick sand matte-treated PET film was superimposed on the pressure-sensitive adhesive layer to be in contact therewith, thus giving an adhesive patch of the present invention.

### Example 4

**[Table 4]**

| Component name | | Solid content /g | Proportion /% |
|---|---|---|---|
| Acrylic polymer | | 2.495 | 49.9 |
| Isopropyl myristate | | 2.5 | 50.0 |
| Magnesium oxide | | 0.005 | 0.1 |
| Ethanol | For dispersing crosslinking agent | 0.25 | - |
| | For adjusting solid content | 1.701 | - |
| Total | | 5 | 100.0 |

As an acrylic polymer, an (acrylic acid-2-ethylhexyl acrylate-vinyl acetate) copolymer (the content of each monomer in the copolymer being 5 mass % for acrylic acid, 70 mass % for 2-ethylhexyl acrylate, and 25 mass % for vinyl acetate) was used. In the above-indicated composition,the acrylic polymer, isopropyl myristate (Spectrum Chemical, NF), and ethanol for adjusting the solids content were mixed, then magnesium oxide (Wako Pure Chemical Industries, Ltd., 0.01 *µ*m) dispersed in ethanol in advance was added thereto, and stirred until the mixture became transparent. The mixture was spread over a silicone-treated surface of an 80 *µ*m-thick polyethylene terephthalate (PET) release film, and heated at 80°C for 15 minutes to remove solvent, while thermally crosslinking to give a 100 *µ*m pressure-sensitive adhesive layer. As a support, a 30 *µ*m-thick sand matte-treated PET film was superimposed on the pressure-sensitive adhesive layer to be in contact therewith, thus giving an adhesive patch of the present invention.

### Comparative Example 1

**[Table 5]**

| Component name | Solid content /g | Proportion /% |
|---|---|---|
| Acrylic polymer | 2.5 | 50.0 |
| Isopropyl myristate | 2.5 | 50.0 |
| Ethanol for adjusting solid content | 1.71 | - |
| Total | 5 | 100.0 |

As an acrylic polymer, an (acrylic acid-2-ethylhexyl acrylate-vinyl acetate) copolymer (the content of each monomer in the copolymer being 5 mass % for acrylic acid, 70 mass % for 2-ethylhexyl acrylate, and 25 mass % for vinyl acetate) was used. In the above-indicated composition, the acrylic polymer, isopropyl myristate (Spectrum Chemical, NF), and ethanol for adjusting the solid content were mixed and stirred to give a uniform mixture, which was spread over a silicone-treated surface of an 80 *µ*m thick polyethylene terephthalate (PET) release film, and solvent was removed by heating at 80°C for 15 minutes, thus giving a 100 *µ*m pressure-sensitive adhesive layer. As a support, a 30 *µ*m-thick sand matte-treated PET film was superimposed on the pressure-sensitive adhesive layer to be in contact therewith, thus giving an adhesive patch of a Comparative Example.

### Comparative Example 2

**[Table 6]**

| Component name | Solid content /g | Proportion /% |
|---|---|---|
| Acrylic polymer | 5 | 100.0 |
| Total | 5 | 100.0 |

As an acrylic polymer, an (acrylic acid-2-ethylhexyl acrylate-vinyl acetate) copolymer (the content of each monomer in the copolymer being 5 mass % for acrylic acid, 70 mass % for 2-ethylhexyl acrylate, and 25 mass % for vinyl acetate) was used and spread over an 80 *µ*m thick silicone-treated surface, and solvent was removed by heating at 80°C for 15 minutes, thus giving a 100 *µ*m pressure-sensitive adhesive layer. As a support, an 80 *µ*m-thick sand matte-treated PET film was superimposed on the pressure-sensitive adhesive layer to be in contact therewith, thus giving an adhesive patch of a comparative example.

### Test Examples

The physical properties (adhesive power and cohesiveness) and corneum-stripping properties of the adhesive patches prepared in Examples 1 to 4 and Comparative Examples 1 and 2 were examined by the methods below.

### Adhesive Power

Measurement method: a 1 cm square test piece was cut out of each patch, and a tack value was measured under the conditions below using a probe tack tester (No. 1216S) manufactured by Rigaku Kogyo.
Probe: Bakelite
Adhesion time: 1 sec
Pulling down speed: 5 mm/sec
Pressure load applied: 20 g

### Evaluation of cohesiveness: plaster remaining on skin

Each adhesive patch was cut into a 25φ test piece and then stuck to an upper arm, 2 hours later it was peeled off, and the plaster remaining on the skin was examined visually.

### Evaluation of cohesiveness: stringiness

Each adhesive patch was cut into a 25φ test piece, and stringiness upon peeling-off of the release film and stringiness upon peeling-off of the pressure-sensitive adhesive layer after being pressed strongly with a finger were examined.

### Corneum stripping test

Each adhesive patch was cut into an 18φ test piece and then stuck to an upper arm, 30 minutes later it was peeled off, and it was immersed in 1 mL of a 0.25% amide black solution (water:acetic acid:methanol = 45:10:45) for 24 hours, thus staining the corneum attached to the patch. After the staining solution was removed, staining solution was washed away by distilled water, the patch was immersed in 2 mL of a decolorizing solution (water:acetic acid:methanol = 8:2:90) for 15 minutes so as to carry out decolorization and remove surplus colorant, and the patch was then air dried. This patch was cut into a 16φ test piece, immersed in 2 mL of elute (0.25 mM NaOH, 0.05 mM EDTA, 1% SDS, 50% ethanol), stirred, and allowed to stand, thus extracting a colorant. The absorbance (630 nm) of this extract was measured.

The results of measurement of the physical properties and the corneum-stripping properties of the adhesive patches prepared in Examples 1 to 4 and Comparative Examples 1 and 2 are shown in Table 7.

From these results, it is clear that the adhesive patches of Examples 1 to 4, which employ the pressure-sensitive adhesive base of the present invention, have both an appropriate adhesive strength and an appropriate cohesive strength, and strip remarkably little corneum when peeled off from the skin.

**[Table 7]**

| | | Probe tack /gF | Cohesiveness | | Corneum-stripping properties (absorbance) |
|---|---|---|---|---|---|
| | | | Plaster remaining | Stringiness | |
| Ex. | 1 | 149.5 | None | None | 0.237 |
| | 2 | 145.0 | None | None | 0.050 |
| | 3 | 204.4 | None | None | 0.146 |
| | 4 | 200.8 | None | None | 0.113 |
| Comp. Ex. | 1 | - | Some | Upon peeling-off of release film | - |
| | 2 | 943.5 | None | None | 0.624 |

## Claims

1. A pressure-sensitive adhesive base comprising components A to C below;
A. a copolymer having as monomer units a carboxyl group-containing monomer and a (meth)acrylic acid ester,
B. one or more type of liquid or semisolid organic compound that is miscible with the copolymer of component A, and
C. at least one type selected from a multivalent metal oxide and a multivalent metal hydroxide,
the total content of component A being 40% to 80% of the total mass of the pressure-sensitive adhesive base, the carboxyl group-containing monomer of the copolymer of component A being 5 to 50 mass %, the total content of component B being at least 10% of the total mass of the pressure-sensitive adhesive base, the total content of component C being 0.1% to 2% of the total mass of the pressure-sensitive adhesive base, and the base containing substantially no water.

2. The pressure-sensitive adhesive base according to Claim 1, wherein it is produced via a step of dispersing and/or dissolving component C in a solvent that does not contain components A and B.

3. A medical adhesive patch comprising a drug and the pressure-sensitive adhesive base according to any one of Claims 1 to 2.

## Patentansprüche

1. Haftklebstoffbasis welche die folgenden Komponenten A bis C umfasst:
A. ein Copolymer welches als Monomereinheiten ein Carboxylgruppen enthaltendes Monomer und einen (Meth)acrylsäureester aufweist,
B. eine oder mehrere Arten von flüssigen oder halbfesten organischen Verbindungen die mit dem Copolymer von Komponente A mischbar sind, und
C. mindestens eine Art, die ausgewählt wird aus einem multivalenten Metalloxyd und einem multivalenten Metallhydroxyd,
wobei der Gesamtgehalt von Komponente A 40% bis 80% der Gesamtmasse der Haftklebstoffbasis beträgt, das Carboxylgruppen enthaltene Monomer des Copolymers von Komponente A 5 bis 50 Masse% beträgt, der Gesamtgehalt von Komponente B mindestens 10% der Gesamtmasse der Haftklebstoffbasis beträgt, der Gesamtgehalt von Komponente C 0.1% bis 2% der Gesamtmasse der Haftklebstoffbasis beträgt und die Basis im Wesentlichen kein Wasser enthält.

2. Haftklebstoffbasis nach Anspruch 1, wobei sie mittels eines Schritts des Dispergierens und/oder Lösens von Komponente C in einem Lösungsmittel hergestellt wird, das die Komponenten A und B nicht enthält.

3. Medizinisches Klebepflaster umfassend ein Arzneimittel und die Haftklebstoffbasis nach einem der Ansprüche 1 bis 2.

## Revendications

1. Base adhésive autocollante comprenant les composants A à C ci-dessous ;
A. un copolymère ayant, comme motifs monomères, un monomère contenant un groupe carboxyle et un ester d'acide (méth)acrylique,
B. un ou plusieurs types de composés organiques liquides ou semi-solides qui sont miscibles avec le copolymère de composant A, et
C. au moins un type choisi parmi un oxyde de métal multivalent et un hydroxyde de métal multivalent,
la teneur totale en composant A étant de 40 % à 80 % de la masse totale de la base adhésive autocollante, le monomère contenant un groupe carboxyle du copolymère de composant A étant de 5 à 50 % en masse, la teneur totale en composant B étant d'au moins 10 % de la masse totale de la base adhésive autocollante, la teneur totale en composant C étant de 0,1 % à 2 % de la masse totale de la base adhésive autocollante, et la base ne contenant sensiblement pas d'eau.

2. Base adhésive autocollante selon la revendication 1, qui est produite par une étape de dispersion et/ou de dissolution du composant C dans un solvant qui ne contient pas les composants A et B.

3. Patch adhésif médical comprenant un médicament et la base adhésive autocollante selon l'une quelconque des revendications 1 à 2.
